# EUROPÄISCHE PATENTSCHRIFT

(11) **EP 1 911 401 B1**
(45) Veröffentlichungstag und Bekanntmachung des Hinweises auf die Patenterteilung: **06.06.2012**
(21) Anmeldenummer: 06021191.9
(22) Anmeldetag: 10.10.2006
(51) Int. Cl.: A61B 6/04, A61G 13/08

(54) **Plattensegment für eine mehrteilige Patientenlagerungsplatte und einen OP-Tisch**
Plate segment for a multi part patient support plate and an operating table
Segment de plaque pour un plateau de support de patient en plusieurs parties et une table d'opération

(43) Veröffentlichungstag der Anmeldung: 16.04.2008
(73) Patentinhaber: TRUMPF Medizin Systeme GmbH + Co. KG, 07318 Saalfeld (DE)
(72) Erfinder: Stolze, Dirk, 07318 Saalfeld (DE); Müller, Ivo, 98739 Piesau (DE)
(74) Vertreter: Karrais, Martin

(56) Entgegenhaltungen:
- EP-A1- 0 677 283
- DE-A1- 4 138 319
- DE-T2- 60 113 409

## Beschreibung

Die Erfindung betrifft ein Plattensegment für eine mehrteilige Patientenlagerungsplatte mit den Merkmalen des Oberbegriffes von Patentanspruch 1.

Ausserdem betrifft die Erfindung eine Patientenlagerungsplatte zur Lagerung eines Patienten mit mehreren relativ zueinander verstellbaren Plattensegmenten sowie einen Operationstisch mit einer Tragsäule, die eine derartige Patientenlagerungsplatte trägt.

Patientenlagerungsplatten dienen der Lagerung eines Patienten insbesondere bei medizinischen Behandlungen und Untersuchungen, beispielsweise während der Untersuchung eines Patienten mittels Röntgenstrahlen. Die Patientenlagerungsplatte ist hierbei mehrteilig ausgebildet und umfasst mehrere Plattensegmente, die relativ zueinander motorisch verstellbar sind. Die Plattensegmente können beispielsweise um quer zur Längsachse der Patientenlagerungsplatte ausgerichtete Schwenkachsen verschwenkt werden. Zum Verstellen der Plattensegmente kommen Elektromotoren zum Einsatz, die an elektrische Verbindungsleitungen, nämlich Signal- und Versorgungsleitungen, angeschlossen sind. Die Plattensegmente umfassen eine Tragplatte, die für Röntgenstrahlen durchlässig ausgebildet ist. Zumindest ein Plattensegment weist zusätzlich mindestens eine elektrische Verbindungsleitung auf, die sich zumindest über einen Teilbereich der Tragplatte erstreckt. Bei einer Röntgenuntersuchung behindern die Verbindungsleitungen den Strahlengang der Röntgenstrahlen. Dies führt zu störenden Abschattungen auf den Röntgenbildern.

Aus DE 601 13 409 T2 ist eine Vorrichtung zur Mammographie- und Impedanzabbildung bekannt, bei der ein Plattensegment mit den Merkmalen des Oberbegriffes von Anspruch 1 zum Einsatz kommt.

Aufgabe der vorliegenden Erfindung ist es, ein Plattensegment der eingangs genannten Art derart weiterzubilden, dass die Verbindungsleitungen auf Röntgenbildern nicht als störende Abschattungen zu erkennen sind.

Diese Aufgabe wird durch ein Plattensegment mit den Merkmalen von Patentanspruch 1 gelöst.

In die Erfindung fließt der Gedanke mit ein, dass durch elektrische Verbindungsleitungen hervorgerufene störende Abschattungen auf Röntgenbildern dadurch vermieden werden können, dass die von der mindestens einen streifenförmig ausgestalteten Leiterbahn hervorgerufene Schwächung der Röntgenstrahlen sich nur geringfügig von der ohnehin durch die Tragplatte hervorgerufenen Schwächung unterscheidet. Die Leiterbahn ist daher sehr dünn aber zugleich relativ breit ausgebildet, so dass der Querschnitt der Leiterbahn mindestens dem sonst erforderlichen Kabelquerschnitt einer in Form eines Elektrokabels ausgestalteten Verbindungsleitung entspricht. Aufgrund der streifenförmigen Ausgestaltung ist die von der Leiterbahn hervorgerufene Abschattung im Röntgenbild entweder überhaupt nicht oder zumindest nicht als störend zu erkennen.

Das erfindungsgemäße Plattensegment zeichnet sich durch eine praktisch über den gesamten Bereich der Tragplatte gleichbleibende Schwächung (Dämpfung) der Röntgenstrahlen aus. Das Material der Tragplatte weist üblicherweise eine verhältnismäßig geringe Schwächung für Röntgenstrahlen aus. Um die etwas stärkere Schwächung, die die Röntgenstrahlen durch die Leiterbahn erfahren, auszugleichen, ist die Leiterbahn in einer Ausnehmung der Tragplatte angeordnet, d. h. die Tragplatte weist in Höhe der Leiterbahn eine geringere Materialstärke auf als in einem Bereich, in dem keine Leiterbahn angeordnet ist. Durch Wahl der Tiefe der Ausnehmung und der Dicke, d. h. der Materialstärke der Leiterbahn, kann somit der Einfluss der Leiterbahn auf die Schwächung der Röntgenstrahlen kompensiert werden.

Gemäß der Erfindung weist das Plattensegment zwei jeweils mit einem verstellbaren Gelenk gekoppelte Elektromotoren auf, die über die mindestens eine streifenförmige Leiterbahn miteinander verbunden sind. Einer der beiden Elektromotoren kann über ein Elektrokabel, das ausserhalb des Plattensegmentes verläuft, an eine Steuer- und Energieversorgungseinrichtung angeschlossen werden, und der andere Elektromotor kann über die mindestens eine Leiterbahn mit dem ersten Elektromotor verbunden sein. Die Gelenke sind mit einem weiteren Plattensegment verbindbar. Mit Hilfe der Elektromotoren können die Gelenke beispielsweise verschwenkt werden, und die Energieversorgung und Signalversorgung des Elektromotors kann über die mindestens eine streifenförmige Leiterbahn erfolgen.

Von Vorteil ist es, wenn die Dicke, d. h. die Materialstärke der Leiterbahn und die Tiefe der Ausnehmung derart gewählt sind, dass Röntgenstrahlen, die das Plattensegment in Höhe der Leiterbahn durchdringen, dieselbe effektive Schwächung erfahren wie Röntgenstrahlen, die das Plattensegment seitlich neben der Leiterbahn durchdringen. Bei einer derartigen Ausgestaltung wird der Einfluss der Leiterbahn auf die Schwächung der Röntgenstrahlen vollkommen kompensiert, so dass auf einem Röntgenbild keinerlei Abschattungen aufgrund der Leiterbahnen erkennbar sind.

Vorzugsweise ist die mindestens eine Leiterbahn aus einem Metall gefertigt, insbesondere aus einem Metall mit geringem Röntgenstrahlen-Schwächungskoeffizienten, z. B. aus einem Aluminiummaterial. Metall zeichnet sich durch eine hohe elektrische Leitfähigkeit aus, so dass elektrische Signale und auch elektrische Versorgungsenergie für einen Elektromotor wirkungsvoll über die Leiterbahn übertragen werden können. Kommt ein Metall mit einem geringen Röntgenstrahlen-Schwächungskoeffizienten zum Einsatz, so kann die Leiterbahn verhältnismäßig dick ausgestaltet sein, ohne dass dies zu einer beträchtlichen Abschattung auf einem Röntgenbild führt. Leiterbahnen aus Aluminium haben sich als besonders vorteilhaft erwiesen.

Die Dicke der mindestens einen Leiterbahn beträgt bei einer bevorzugten Ausführungsform maximal 0,5 mm. Insbesondere kann vorgesehen sein, dass die Dicke der mindestens einen Leiterbahn ca. 0,1 mm beträgt.

Die Breite der mindestens einen Leiterbahn beträgt bei einer bevorzugten Ausführungsform ein Mehrfaches von deren Dicke, insbesondere mindestens das Zehnfache, zum Beispiel das Fünfzig- bis Einhundertfache. Eine große Breite hat den Vorteil, dass die Leiterbahn einen großen Querschnitt aufweisen kann, so dass elektrische Signale und elektrische Versorgungsenergie mit nur sehr geringen Verlusten übertragen werden können.

Die Tragplatte ist vorzugsweise aus einem Kunststoffmaterial gefertigt, insbesondere aus einem faserverstärkten Kunststoffmaterial. Es kann beispielsweise vorgesehen sein, dass die Tragplatte aus einem mittels Zellulosefasern verstärkten Kunststoffmaterial gefertigt ist. So kann zum Beispiel ein Kunststoffmaterial auf Basis thermohärtender Harze zum Einsatz kommen, das homogen mit Zellulosefasern verstärkt ist.

Günstig ist es, wenn das Plattensegment mehrere streifenförmige elektrische Verbindungsleitungen in Form von Leiterbahnen aufweist, die nebeneinander angeordnet sind und sich gegenseitig nicht überlappen. Die überlappungsfreie Anordnung der Leiterbahnen stellt auf konstruktiv einfache Weise sicher, dass Röntgenstrahlen, die das Plattensegment durchgreifen, eine möglichst gleichbleibende Schwächung erfahren. Grundsätzlich ist es jedoch auch möglich, mehrere Leiterbahnen einander überlappend anzuordnen. Es ist dann vorteilhaft, die Materialstärke der Tragplatte im Überlappungsbereich der Leiterbahnen besonders gering zu wählen, so dass die stärkere Schwächung, die die Röntgenstrahlen im Überlappungsbereich der Leiterbahnen erfahren, durch eine besonders geringe Schwächung im Bereich der Tragplatte oberhalb und/oder unterhalb des Überlappungsbereiches kompensiert wird.

Bei einer besonders bevorzugten Ausgestaltung des erfindungsgemäßen Plattensegmentes ist die Schwächung von Röntgenstrahlung, die das Plattensegment im Bereich zwischen zwei Leiterbahnen durchdringen, ebenso groß wie die Schwächung, die die Röntgenstrahlen beim Durchdringen des Plattensegmentes in Höhe einer Leiterbahn erfahren. Dies kann beispielsweise dadurch erzielt werden, dass im Bereich zwischen zwei Leiterbahnen ein elektrisch nicht leitendes, aber Röntgenstrahlen in gleichem Maße wie die Leiterbahnen schwächendes Material angeordnet ist. Alternativ kann vorgesehen sein, dass die Tragplatte im Bereich zwischen zwei Leiterbahnen eine größere Dicke (Materialstärke) aufweist als im Bereich der Leiterbahnen.

Bei einer vorteilhaften Ausführungsform sind die Leiterbahnen mit fluchtend zueinander angeordneten Rändern nebeneinander angeordnet. Dadurch kann ein die Röntgenstrahlen in gleichem Maße wie die Leiterbahnen schwächendes Material zwischen den Leiterbahnen entfallen. Vielmehr bilden die Leiterbahnen in ihrer Gesamtheit eine Fläche mit gleichbleibender Dicke aus, so dass die Röntgenstrahlen über den Bereich der Leiterbahnen eine gleichbleibende Schwächung erfahren.

Vorzugsweise sind die Leiterbahnen in unterschiedlichen Ebenen angeordnet. Beispielsweise kann vorgesehen sein, dass mindestens eine Leiterbahn in einer ersten Ebene und mindestens eine zweite Leiterbahn in einer zweiten Ebene angeordnet ist. Benachbarte Leiterbahnen sind mit ihren einander zugewandten Rändern fluchtend ausgerichtet und jeweils in einer Ausnehmung der Tragplatte angeordnet.

Günstig ist es, wenn die Tragplatte als Mehrschichtplatte ausgestaltet ist, wobei mindestens zwei Schichten Ausnehmungen aufweisen, in denen jeweils eine Leiterbahn angeordnet ist. Die Tragplatte kann beispielsweise eine obere und eine untere Tragplattenschicht umfassen, wobei die obere Tragplattenschicht unterseitig und die untere Tragplattenschicht oberseitig Ausnehmen aufweist, in denen jeweils eine Leiterbahn angeordnet ist.

Die Ausnehmungen der Tragplatte sind vorzugsweise als U-förmige Nuten ausgebildet.

Die Leiterbahnen können unterschiedliche Breiten aufweisen. So können beispielsweise Leiterbahnen, über die elektrische Signale übertragen werden, eine geringere Breite aufweisen als Leiterbahnen, über die ein Elektromotor mit elektrischer Energie versorgt wird.

Von besonderem Vorteil ist es, wenn die Leiterbahnen jeweils von einem elektrischen Isolationsmaterial umhüllt sind. Als Isolationsmaterial kann beispielsweise ein Polyester-Material zum Einsatz kommen. Mittels der Umhüllung kann auf konstruktiv einfache Weise ein elektrischer Kurzschluss zwischen einander benachbarten Leiterbahnen vermieden werden.

Wie bereits erläutert, betrifft die Erfindung nicht nur ein Plattensegment der voranstehend genannten Art sondern auch eine Patientenlagerungsplatte mit mehreren relativ zueinander verstellbaren Plattensegmenten, wobei mindestens ein Plattensegment gemäß den voranstehenden Erläuterungen ausgebildet ist.

Ausserdem betrifft die Erfindung einen Operationstisch mit einer Tragsäule, die eine derartige Patientenlagerungsplatte trägt. Günstig ist es, wenn die Patientenlagerungsplatte mit der Tragplatte lösbar verbindbar ist. Dies gibt die Möglichkeit, die Patientenlagerungsplatte von der Tragsäule zu lösen und beispielsweise auf einen Trolley aufzusetzen.

Die nachfolgende Beschreibung zweier bevorzugter Ausführungsformen der Erfindung dient im Zusammenhang mit der Zeichnung der näheren Erläuterung. Es zeigen:
- Figur 1:: eine schematische Seitenansicht eines Operationstisches mit einer mehrteiligen Patientenlagerungsplatte;
- Figur 2:: eine Draufsicht auf den Operationstisch aus Figur 1;
- Figur 3:: eine vergrößerte Draufsicht auf eine erste Ausführungsform eines erfindungsgemäßen Plattensegments der Patientenlagerungsplatte aus Figur 1;
- Figur 4:: eine Schnittansicht des Plattensegmentes aus Figur 3 entlang der Linie 4-4 und
- Figur 5:: eine Schnittansicht einer zweiten Ausführungsform eines erfindungsgemäßen Plattensegmentes entsprechend Figur 4.

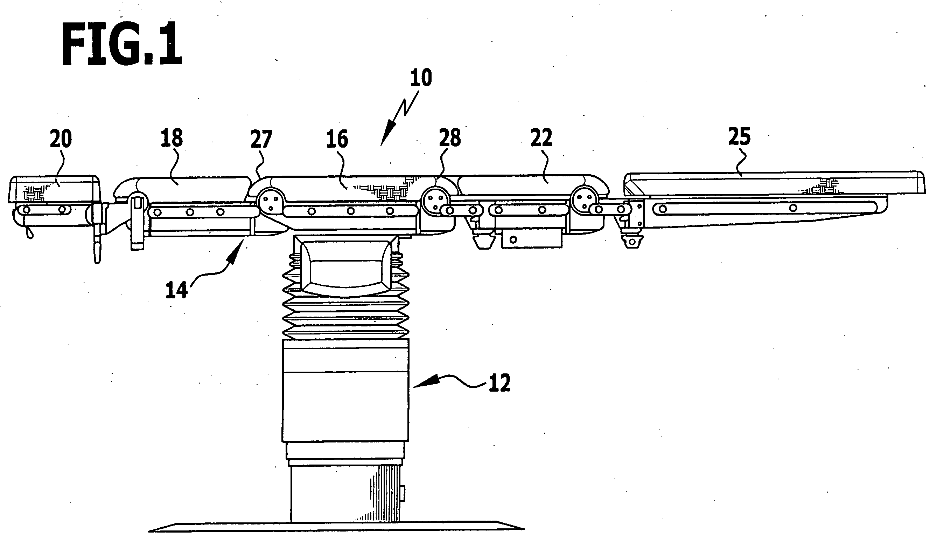

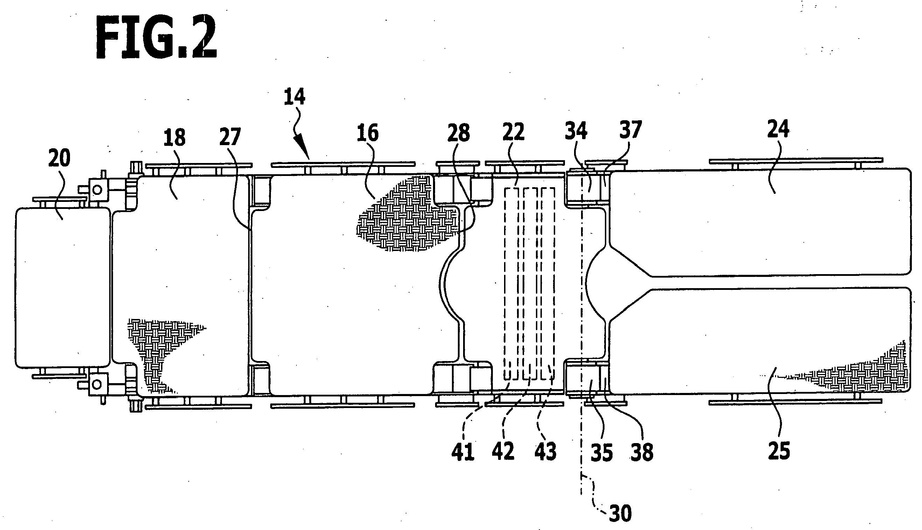

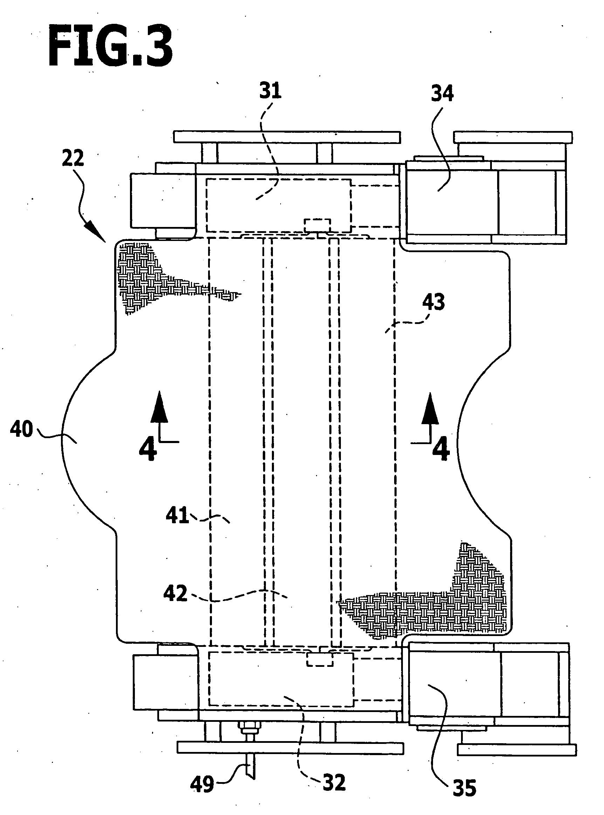

In den Figuren 1 und 2 ist schematisch ein Operationstisch 10 dargestellt mit einer höhenverstellbaren Tragsäule 12, auf die eine Patientenlagerungsplatte 14 aufgesetzt ist. Die Patientenlagerungsplatte 14 umfasst mehrere, relativ zueinander verstellbare Plattensegmente in Form eines Basissegmentes 16, eines oberen Rückensegments 18 und eines Kopfsegments 20 sowie eines Beckensegments 22 und eines linken und eines rechten Beinsegments 24 bzw. 25. Das obere Rückensegment 18 schließt sich in Längsrichtung der Patientenlagerungsplatte 14 an eine erste Seite 27 des Basissegments 16 an, und an das obere Rückensegment 18 schließt sich das Kopfsegment 20 an. An die dem oberen Rückensegment 18 abgewandte zweite Seite 28 des Basissegments 16 schließt sich das Beckensegment 22 an, das auf seiner dem Basissegment 16 abgewandten Seite die beiden Beinsegmente 24 und 25 trägt. Die Beinsegmente 24 und 25 sind um eine gemeinsame, senkrecht zur Längsrichtung der Patientenlagerungsplatte 14 ausgerichtete Schwenkachse 30 verschwenkbar. Hierzu weist das Beckensegment 22 einen ersten Elektromotor 31 und einen zweiten Elektromotor 32 auf. Dies wird aus Figur 3 deutlich, die eine vergrößerte Draufsicht auf das Beckensegment 22 zeigt. Die beiden Elektromotoren 31 und 32 sind jeweils mit einem verstellbaren Gelenk 34 bzw. 35 gekoppelt. Die Gelenke 34 und 35 können von den Elektromotoren 31 und 32 um die Schwenkachse 30 verschwenkt werden und nehmen jeweils ein Anschlussteil 37 bzw. 38 der Beinsegmente 24 und 25 auf. Über die Anschlussteile 37 und 38 können die Beinsegmente 24 und 25 entsprechend den Gelenken 34 und 35 verschwenkt werden.

Die beiden Elektromotoren 31 und 32 sind an einer Tragplatte 40 des Beckensegments 22 angeordnet und über drei Leiterbahnen 41, 42, 43 elektrisch miteinander verbunden. Die Leiterbahnen 41, 42, 43 sind parallel und im Abstand zueinander angeordnet und verlaufen quer zur Längsrichtung der Patientenlagerungsplatte 14. Sie sind jeweils in einer Ausnehmung der Tragplatte 40 in Form einer unterseitig angeordneten U-förmigen Nut 45, 46 bzw. 47 angeordnet. An den zweiten Elektromotor 30 ist außerdem ein mehradriges Elektrokabel 49 angeschlossen, das ausserhalb der Patientenlagerungsplatte 14 verläuft und an eine in die Tragsäule 12 integrierte, an sich bekannte und deshalb in der Zeichnung nicht dargestellte Steuer- und Energieversorgungseinheit angeschlossen werden kann. Von der Steuer- und Energieversorgungseinheit können über das Elektrokabel 49 Steuersignale sowie Elektroenergie an den zweiten Elektromotor 32 übertragen werden, von dem aus die Signale und die Energie über die Leiterbahnen 41, 42, 43 dem ersten Elektromotor 31 zugeführt werden können.

Die Tragplatte 40 ist aus einem Kunststoffmaterial gefertigt, nämlich aus einem Material auf Basis thermohärtender Harze, das homogen mit Zellulosefasern verstärkt ist. Die Leiterbahnen 41, 42 und 43 sind aus einem Aluminiummaterial hergestellt. Die Dicke der Leiterbahnen beträgt ca. 0,1 mm, wohingegen ihre Breite ein Mehrfaches ihrer Dicke beträgt, beispielsweise ca. 5 mm.

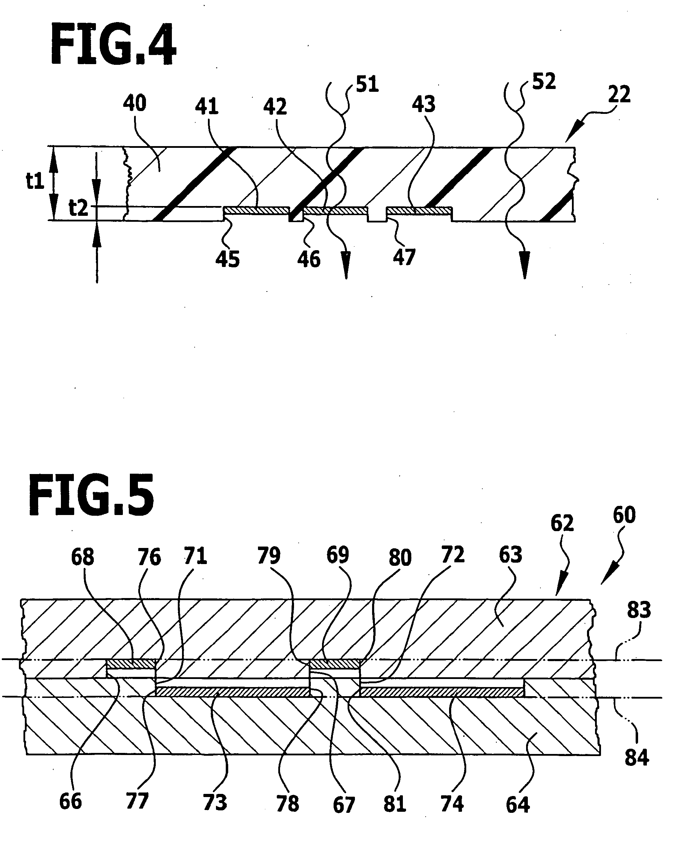

Wie insbesondere aus Figur 4 deutlich wird, ist die Materialstärke der Tragplatte aufgrund der eingeformten Nuten 45, 46, 47 in Höhe der Leiterbahnen 41, 42, 43 geringer als im Bereich zwischen den Leiterbahnen 41, 42,43 und seitlich neben den Leiterbahnen 41, 42, 43. Seitlich neben den Leiterbahnen und zwischen den Leiterbahnen weist die Tragplatte 40 eine Dicke t1 auf und die Tiefe der Nut beträgt t2. Die Nuttiefe t2 und die Gesamtdicke t1 sind derart gewählt, dass Röntgenstrahlen, die das Beckensegment 22 in Höhe der Leiterbahnen 41, 42, 43 durchdringen, praktisch dieselbe Schwächung erfahren wie Röntgenstrahlen, die das Beckensegment 22 in einem Bereich seitlich neben den Leiterbahnen 41, 42, 43 durchdringen. Die entsprechenden Röntgenstrahlen sind in Figur 4 schematisch durch die Pfeile 51 und 52 symbolisiert.

Wird ein auf der Patientenlagerungsplatte 14 ruhender Patient im Bereich des Beckensegments 22 einer röntgendiagnostischen Untersuchung unterworfen, so zeigt das Röntgenbild keine Abschattungen, die von den Leiterbahnen 41, 42 oder 43 hervorgerufen werden. Vielmehr wird aufgrund der Wahl der Dicke t1 und der Nuttiefe t2 sowie der Dicke der Leiterbahnen 41, 42, 43 über den gesamten Bereich der Tragplatte 40 eine gleichbleibende Röntgenschwächung, d. h. Dämpfung der Röntgenstrahlen, sichergestellt.

In Figur 5 ist eine alternative Ausgestaltung eines insgesamt mit dem Bezugszeichen 60 belegten Beckensegments dargestellt. Dieses ist weitgehend identisch ausgebildet wie das voranstehend unter Bezugnahme auf die Figuren 1, 2, 3 und 4 beschriebene Beckensegment 22. Das Beckensegment 60 weist im Unterschied zum Beckensegment 22 eine Tragplatte 62 auf, die als Mehrschichtplatte ausgestaltet ist und eine obere Tragplattenschicht 63 und eine untere Tragplattenschicht 64 umfasst. Die obere Tragplattenschicht 63 weist unterseitig zwei Nuten 66, 67 auf, die jeweils U-förmig ausgebildet und parallel zueinander angeordnet sind. In den Nuten 66 und 67 ist jeweils eine streifenförmige Leiterbahn 68 bzw. 69 angeordnet, die von einem in der Zeichnung nicht dargestellten elektrischen Isolationsmaterial, beispielsweise einem Polyestermaterial, umhüllt ist.

Die untere Tragplattenschicht 64 weist oberseitig, also der oberen Tragplattenschicht 63 zugewandt, im Abstand zueinander zwei U-förmige Nuten 71 und 72 auf, in denen jeweils eine Leiterbahn 63 bzw. 64 angeordnet ist, die von einem in der Zeichnung nicht dargestellten elektrischen Isolationsmaterial, vorzugsweise einem Polyestermaterial, umhüllt ist. Wie aus Figur 5 deutlich wird, sind die Leiterbahnen 68, 69, 73 und 74 mit fluchtend zueinander angeordneten Rändern nebeneinander angeordnet, d. h. der der Leiterbahn 73 zugewandte Rand 76 der Leiterbahn 68 fluchtet mit dem Rand 77 der Leiterbahn 73, der der Leiterbahn 69 zugewandte Rand 78 der Leiterbahn 73 fluchtet mit dem Rand 79 der Leiterbahn 69 und der der Leiterbahn 74 zugewandte Rand 80 der Leiterbahn 69 fluchtet mit dem Rand 81 der Leiterbahn 74. Die beiden Leiterbahnen 68 und 69 sind hierbei in einer ersten Ebene 83 angeordnet und die beiden Leiterbahnen 73 und 74 sind in einer zweiten Ebene 84 angeordnet, die parallel zur ersten Ebene 83 verläuft.

Aufgrund ihrer miteinander fluchtenden Ränder schließen die Leiterbahnen 68, 73, 69 und 74 quer zur Durchstrahlungsrichtung der Röntgenstrahlen unmittelbar aneinander an, ohne miteinander zu verlappen. Dies hat zur Folge, dass die Leiterbahnen 68, 73, 69 und 74 bezogen auf Röntgenstrahlen, die das Beckensegment 60 durchdringen, eine homogene Fläche ausbilden.

Die Materialstärke der oberen Tragplattenschicht 63 und die Nuttiefe der Nuten 66 und 67 sind entsprechend der Ausgestaltung der voranstehend unter Bezugnahme auf die Figuren 1 bis 4 erläuterten Tragplatte 40 ausgebildet. Die Materialstärke der oberen Tragplattenschicht 63 ist seitlich neben den Leiterbahnen 68 und 69 also so gewählt, dass durchdringende Röntgenstrahlen eine gleichbleibende Schwächung erfahren unabhängig davon, ob sie lediglich das Material der oberen Tragplattenschicht 63 durchdringen oder auch die Leiterbahnen 68 oder 69. In entsprechender Weise sind auch die Materialstärke und die Nuttiefe der unteren Tragplattenschicht 64 gewählt. Dies hat zur Folge, dass auch bei der in Figur 5 dargestellten Ausführungsform Röntgenstrahlen eine gleichmäßige Schwächung erfahren unabhängig davon, ob sie auf eine Leiterbahn treffen oder nicht. Auf einem Röntgenbild werden somit durch die Leiterbahnen 68, 69 und 73, 74 keine störenden Abschattungen erzeugt.

## Patentansprüche

1. Plattensegment für eine mehrteilige Patientenlagerungsplatte, wobei das Plattensegment (22; 60) eine röntgenstrahlungsdurchlässige Tragplatte (40; 62) aufweist sowie mindestens eine elektrische Verbindungsleitung, die sich zumindest über einen Teilbereich der Tragplatte (40; 62) erstreckt, und wobei die mindestens eine Verbindungsleitung als streifenförmige Leiterbahn (41, 42, 43; 68, 69, 73, 74) ausgestaltet ist, die in einer Ausnehmung (45, 46, 47; 66, 67, 71, 72) der Tragplatte (40; 62) angeordnet ist, **dadurch gekennzeichnet, dass** das Plattensegment (22; 60) zwei jeweils mit einem verstellbaren Gelenk (34, 35) gekoppelte Elektromotoren (31, 32) aufweist, die über die mindestens eine streifenförmige Leiterbahn (41, 42, 43; 68, 69, 73, 74) miteinander verbunden sind, wobei die Gelenke (34, 35) mit einem weiteren Plattensegment (24, 25) verbindbar sind

2. Plattensegment nach Anspruch 1, **dadurch gekennzeichnet, dass** die Dicke der mindestens einen Leiterbahn (41, 42, 43; 68, 69, 73, 74) und die Tiefe der Ausnehmung (45, 46, 47; 66, 67, 71, 72) derart gewählt sind, dass Röntgenstrahlen, die das Plattensegment (22; 60) in Höhe der Leiterbahn (41, 42, 43; 68, 69, 73, 74) durchdringen dieselbe effektive Schwächung erfahren wie Röntgenstrahlen, die das Plattensegment (22; 60) seitlich neben der mindestens einen Leiterbahn (41, 42, 43; 68, 69, 73, 74) durchdringen.

3. Plattensegment nach einem der voranstehenden Ansprüche, **dadurch gekennzeichnet, dass** die mindestens eine Leiterbahn (41, 42, 43; 68, 69, 73, 74) aus einem Metall gefertigt ist.

4. Plattensegment nach einem der voranstehenden Ansprüche, **dadurch gekennzeichnet, dass** die mindestens eine Leiterbahn (41, 42, 43; 68, 69, 73, 74) aus einem Aluminiummaterial gefertigt ist.

5. Plattensegment nach einem der voranstehenden Ansprüche, **dadurch gekennzeichnet, dass** die Dicke der mindestens einen Leiterbahn (41, 42, 43; 68, 69, 73, 74) maximal 0,5 mm beträgt.

6. Plattensegment nach Anspruch 5, **dadurch gekennzeichnet, dass** die Dicke der mindestens einen Leiterbahn (41, 42, 43; 68, 69, 73, 74) ca. 0,1 mm beträgt.

7. Plattensegment nach einem der voranstehenden Ansprüche, **dadurch gekennzeichnet, dass** die Breite der mindestens einen Leiterbahn (41, 42, 43; 68, 69, 73, 74) ein Mehrfaches von deren Dicke beträgt.

8. Plattensegment nach einem der voranstehenden Ansprüche, **dadurch gekennzeichnet, dass** die Tragplatte (40; 62) aus einem Kunststoffmaterial gefertigt ist, insbesondere aus einem faserverstärkten Kunststoffmaterial.

9. Plattensegment nach einem der voranstehenden Ansprüche, **dadurch gekennzeichnet, dass** das Plattensegment (22; 60) mehrere streifenförmige elektrische Verbindungsleitungen in Form von Leiterbahnen (41, 42, 43; 68, 69, 73, 74) aufweist, die nebeneinander angeordnet sind und sich gegenseitig nicht überlappen.

10. Plattensegment nach Anspruch 9, **dadurch gekennzeichnet, dass** die Schwächung von Röntgenstrahlen, die das Plattensegment (22; 60) im Bereich zwischen zwei Leiterbahnen durchdringen, ebenso groß ist wie die Schwächung, die die Röntgenstrahlen beim Durchdringen des Plattensegmentes (22; 60) in Höhe einer Leiterbahn (41, 42, 43; 68, 69, 73, 74) erfahren.

11. Plattensegment nach Anspruch 9 oder 10, **dadurch gekennzeichnet, dass** die Leiterbahnen (68, 73, 69, 74) mit fluchtend zueinander angeordneten Rändern (76, 77; 78, 79; 80, 81) angeordnet sind.

12. Plattensegment nach Anspruch 9, 10 oder 11, **dadurch gekennzeichnet, dass** die Leiterbahnen (68, 73, 69, 74) in mehreren Ebenen (83, 84) angeordnet sind.

13. Plattensegment nach einem der Ansprüche 9 bis 12, **dadurch gekennzeichnet, dass** die Tragplatte (62) als Mehrschichtplatte ausgestaltet ist, wobei zumindest zwei Tragplattenschichten (63, 64) Ausnehmungen (66, 67; 71, 72) aufweisen, in denen jeweils eine Leiterbahn (68, 69; 73, 74) angeordnet ist.

14. Plattensegment nach einem der Ansprüche 9 bis 13, **dadurch gekennzeichnet, dass** die Leiterbahnen (68, 69, 73, 74) unterschiedliche Breiten aufweisen.

15. Plattensegment nach einem der Ansprüche 9 bis 14, **dadurch gekennzeichnet, dass** die Leiterbahnen (68, 69, 73, 74) jeweils von einem elektrischen Isolationsmaterial umhüllt sind.

16. Patientenlagerungsplatte zur Lagerung eines Patienten mit mehreren relativ zueinander verstellbaren Plattensegmenten (16, 18, 20, 22, 24, 25), wobei mindestens ein Plattensegment (22) nach einem der voranstehenden Ansprüche ausgestaltet ist.

17. Operationstisch mit einer Tragsäule (12), die eine Patientenlagerungsplatte (14) nach Anspruch 16 trägt.

## Claims

1. Panel segment for a multipart patient supporting panel, the panel segment (22; 60) comprising a radiotransparent support panel (40; 62) and at least one electrical connecting line, which extends at least over a partial region of the support panel (40; 62), and the at least one connecting line being configured as a strip conductor (41, 42, 43; 68, 69, 73, 74) that is disposed in a recess (45, 46, 47; 66, 67, 71, 72) in the support panel (40; 62), **characterized in that** the panel segment (22; 60) has two electric motors (31, 32), each of which is coupled to an adjustable joint (34, 35), and which are connected to each other by means of the at least one strip conductor (41, 42, 43; 68, 69, 73, 74), the joints (34, 35) being connectable to a further panel segment (24, 25).

2. Panel segment according to Claim 1, **characterized in that** the thickness of the at least one conductor (41, 42, 43; 68, 69, 73, 74) and the depth of the recess (45, 46, 47; 66, 67, 71, 72) are chosen such that x-rays that penetrate the panel segment (22; 60) at the level of the conductor (41, 42, 43; 68, 69, 73, 74) undergo the same effective attenuation as x-rays that penetrate the panel segment (22; 60) laterally alongside the at least one conductor (41, 42, 43; 68, 69, 73, 74).

3. Panel segment according to one of the preceding claims, **characterized in that** the at least one conductor (41, 42, 43; 68, 69, 73, 74) is produced from a metal.

4. Panel segment according to one of the preceding claims, **characterized in that** the at least one conductor (41, 42, 43; 68, 69, 73, 74) is produced from an aluminum material.

5. Panel segment according to one of the preceding claims, **characterized in that** the thickness of the at least one conductor (41, 42, 43; 68, 69, 73, 74) is at most 0.5 mm.

6. Panel segment according to Claim 5, **characterized in that** the thickness of the at least one conductor (41, 42, 43; 68, 69, 73, 74) is about 0.1 mm.

7. Panel segment according to one of the preceding claims, **characterized in that** the width of the at least one conductor (41, 42, 43; 68, 69, 73, 74) is a multiple of its thickness.

8. Panel segment according to one of the preceding claims, **characterized in that** the support panel (40; 62) is produced from a plastics material, in particular from a fiber-reinforced plastics material.

9. Panel segment according to one of the preceding claims, **characterized in that** the panel segment (22; 60) has a number of strip-shaped electrical connecting lines in the form of conductors (41, 42, 43; 68, 69, 73, 74) that are disposed alongside one another and do not overlap one another.

10. Panel segment according to Claim 9, **characterized in that** the attenuation of x-rays that penetrate the panel segment (22; 60) in the region between two conductors is just as great as the attenuation that the x-rays experience when they penetrate the panel segment (22; 60) at the level of a conductor (41, 42, 43; 68, 69, 73, 74).

11. Panel segment according to Claim 9 or 10, **characterized in that** the conductors (68, 73, 69, 74) are disposed with edges (76, 77; 78, 79; 80, 81) aligned with one another.

12. Panel segment according to Claim 9, 10 or 11, **characterized in that** the conductors (68, 73, 69, 74) are disposed in a number of planes (83, 84).

13. Panel segment according to one of Claims 9 to 12, **characterized in that** the support panel (62) is configured as a multilayer panel, at least two support panel layers (63, 64) having recesses (66, 67; 71, 72) in each of which a conductor (68, 69; 73, 74) is disposed.

14. Panel segment according to one of Claims 9 to 13, **characterized in that** the conductors (68, 69, 73, 74) have different widths.

15. Panel segment according to one of Claims 9 to 14, **characterized in that** the conductors (68, 69, 73, 74) are respectively enclosed by an electrically insulating material.

16. Patient supporting panel for supporting a patient, with a number of panel segments (16, 18, 20, 22, 24, 25) that are adjustable in relation to one another, at least one panel segment (22) being configured according to one of the preceding claims.

17. Operating table with a support column (12), which supports a patient supporting panel (14) according to Claim 16.

## Revendications

1. Segment de plateau pour un plateau de support de patient à plusieurs éléments, ledit segment de plateau (22 ; 60) comportant un plateau porteur radiotransparent (40 ; 62) ainsi qu'au moins une ligne de connexion électrique qui s'étend au moins sur une zone partielle du plateau porteur (40 ; 62), et la ou les lignes de connexion étant réalisées comme pistes conductrices en forme de bandes (41, 42, 43 ; 68, 69, 73, 74) disposées dans un évidement (45, 46, 47 ; 66, 67, 71, 72) du plateau porteur (40 ; 62), **caractérisé en ce que** le segment de plateau (22 ; 60) comporte deux moteurs électriques (31, 32) accouplés chacun à une articulation réglable (34, 35), lesquels sont reliés l'un à l'autre par la ou les pistes conductrices en forme de bandes (41, 42, 43 ; 68, 69, 73, 74), les articulations (34, 35) pouvant être raccordées à un autre segment de plateau (24, 25).

2. Segment de plateau selon la revendication 1, **caractérisé en ce que** l'épaisseur de la piste ou des pistes conductrices (41, 42, 43; 68, 69, 73, 74) et la profondeur de l'évidement (45, 46, 47; 66, 67, 71, 72) sont choisies de telle manière que des rayons X traversant le segment de plateau (22 ; 60) au niveau de la piste conductrice (41, 42, 43 ; 68, 69, 73, 74) subissent le même affaiblissement effectif que des rayons X traversant latéralement le segment de plateau (22 ; 60), à côté de la piste ou des pistes conductrices (41, 42, 43 ; 68, 69, 73, 74).

3. Segment de plateau selon l'une des revendications précédentes, **caractérisé en ce que** la ou les pistes conductrices (41, 42, 43 ; 68, 69, 73, 74) sont constituées d'un métal.

4. Segment de plateau selon l'une des revendications précédentes, **caractérisé en ce que** la ou les pistes conductrices (41, 42, 43 ; 68, 69, 73, 74) sont constituées d'un matériau aluminium.

5. Segment de plateau selon l'une des revendications précédentes, **caractérisé en ce que** l'épaisseur de la piste ou des pistes conductrices (41, 42, 43 ; 68, 69, 73, 74) est au maximum de 0,5 mm.

6. Segment de plateau selon la revendication 5, **caractérisé en ce que** l'épaisseur de la piste ou des pistes conductrices (41, 42, 43 ; 68, 69, 73, 74) est d'environ 0,1 mm.

7. Segment de plateau selon l'une des revendications précédentes, **caractérisé en ce que** la largeur de la piste ou des pistes conductrices (41, 42, 43 ; 68, 69, 73, 74) est un multiple de son épaisseur.

8. Segment de plateau selon l'une des revendications précédentes, **caractérisé en ce que** le plateau porteur (40 ; 62) est constitué d'une matière plastique, en particulier d'une matière plastique renforcée par des fibres.

9. Segment de plateau selon l'une des revendications précédentes, **caractérisé en ce que** le segment de plateau (22 ; 60) comporte plusieurs lignes de connexion électriques en forme de bandes sous forme de pistes conductrices (41, 42, 43 ; 68, 69, 73, 74), lesquelles sont disposées côte à côte et ne se chevauchent pas.

10. Segment de plateau selon la revendication 9, **caractérisé en ce que** l'affaiblissement des rayons X qui traversent le segment de plateau (22 ; 60) dans la zone comprise entre deux pistes conductrices est équivalent à l'affaiblissement subi par les rayons X traversant le segment de plateau (22 ; 60) au niveau d'une piste conductrice (41, 42, 43 ; 68, 69, 73, 74).

11. Segment de plateau selon la revendication 9 ou 10, **caractérisé en ce que** les pistes conductrices (68, 73, 69, 74) sont disposées avec des bords (76, 77 ; 78, 79 ; 80, 81) alignés entre eux.

12. Segment de plateau selon la revendication 9, 10 ou 11, **caractérisé en ce que** les pistes conductrices (68, 73, 69, 74) sont disposées sur plusieurs plans (83, 84).

13. Segment de plateau selon l'une des revendications 9 à 12, **caractérisé en ce que** le plateau porteur (62) est prévu comme plateau stratifié, au moins deux couches de plateau porteur (63, 64) comportant des évidements (66, 67 ; 71, 72) où est respectivement disposée une piste conductrice (68, 69 ; 73, 74).

14. Segment de plateau selon l'une des revendications 9 à 13, **caractérisé en ce que** les pistes conductrices (68, 69, 73, 74) sont de largeurs différentes.

15. Segment de plateau selon l'une des revendications 9 à 14, **caractérisé en ce que** les pistes conductrices (68, 69, 73, 74) sont respectivement enrobées d'un matériau isolant électrique.

16. Plateau de support de patient destiné à supporter un patient et comportant plusieurs segments de plateau (16, 18, 20, 22, 24, 25) déplaçables relativement entre eux, au moins un segment de plateau (22) étant réalisé selon l'une des revendications précédentes.

17. Table d'opération avec une colonne porteuse (12) supportant un plateau de support de patient (14) selon la revendication 16.
